# EUROPEAN PATENT SPECIFICATION

(11) **EP 3 087 967 B1**
(45) Date of publication and mention of the grant of the patent: **27.02.2019**
(21) Application number: 16160267.7
(22) Date of filing: 15.03.2016
(51) Int. Cl.: A61H 1/02, A61H 3/00

(54) **ADJUSTABLE POSITION PIVOT FOR MEDICAL ASSIST DEVICE**
GELENK MIT EINSTELLBARER POSITION FÜR MEDIZINISCHE TRAGEVORRICHTUNG
PIVOT À POSITION RÉGLABLE POUR DISPOSITIF D'ASSISTANCE MÉDICALE

(30) Priority: 29.04.2015 US 201514699390
(43) Date of publication of application: 02.11.2016
(73) Proprietor: Steering Solutions IP Holding Corporation, Saginaw, MI 48601 (US)
(72) Inventor: Simon, Daniel, C., Freeland, MI 48623 (US); Palmer, Travis, L., Frankenmuth, MI 48734 (US)
(74) Representative: Manitz Finsterwald Patentanwälte PartmbB

(56) References cited:
- WO-A1-2012/070244
- WO-A1-2014/093470
- JP-A- 2012 217 746
- KR-B1- 100 731 899
- TW-A- 201 330 843

## Description

### FIELD OF THE INVENTION

The following description relates to medical assist devices and, more specifically, to an adjustable exoskeleton device.

### BACKGROUND

Some exoskeleton devices may be used to assist medical patients with one or more movements. For example, exoskeleton devices may be provided for the arms or legs of a user. Where a user has full use of the limb supported by the exoskeleton device, it may be used to enhance natural abilities such as load carrying. Where the user has impaired use of the limb supported by the exoskeleton device, it may be used for rehabilitative purposes or to replicate a full physical function. Such devices may be powered by one or more motors coupled to gears or pulleys configured to move a user's limb in a desired motion, such as walking.

However, some exoskeleton devices are not adjustable and may not fit some users due to size constraints. Accordingly, it is desirable to provide an exoskeleton device that is adjustable to fit different sized users.

KR 10-0731899 discloses an adjustable medical assist device in accordance with the preamble of claim 1.

WO 2012/070244 teaches an adjustable medical assist device in which a leg support is configured to pivot about an axis that extends parallel to a support arm of a support frame.

CN 201330843, JP 2012-217746 and WO 2014/093470 show examples of prior art human exoskeletons configured to be worn about a user's hips and/or legs.

### SUMMARY OF THE INVENTION

Claim 1 provides an adjustable medical assist device in accordance with the present invention and claim 7 provides a method for assembling an adjustable medical assist device in accordance with the present invention. The following discussion is merely provided for better understanding the present disclosure.

An adjustable joint actuator assembly for a medical assist device can include a support arm extending along a longitudinal axis, a gearbox rotatably coupled to the support arm about the longitudinal axis and slidable along the longitudinal axis, and a locking mechanism inserted over a portion of the support arm and the gearbox to prevent movement of the gearbox relative to the support arm along the longitudinal axis. The locking mechanism can be configured for removal to enable movement of the gearbox relative to the support arm along the longitudinal axis.

These and other advantages and features will become more apparent from the following description taken in conjunction with the drawings.

### BRIEF DESCRIPTION OF THE DRAWINGS

The subject matter which is regarded as the invention is particularly pointed out and distinctly claimed in the claims at the conclusion of the specification. The foregoing and other features, and advantages of the invention are apparent from the following detailed description taken in conjunction with the accompanying drawings in which:
FIG. 1 is a perspective view of an exemplary adjustable medical assist device;
FIG. 2 is perspective view of an exemplary gearbox assembly of the device shown in FIG. 1 before assembly;
FIG. 3 is a perspective view of the gearbox assembly shown in FIG. 2 after assembly; and
FIG. 4 is a cross-sectional view of the gearbox assembly shown in FIG. 3 and taken along line 4-4.

### DETAILED DESCRIPTION

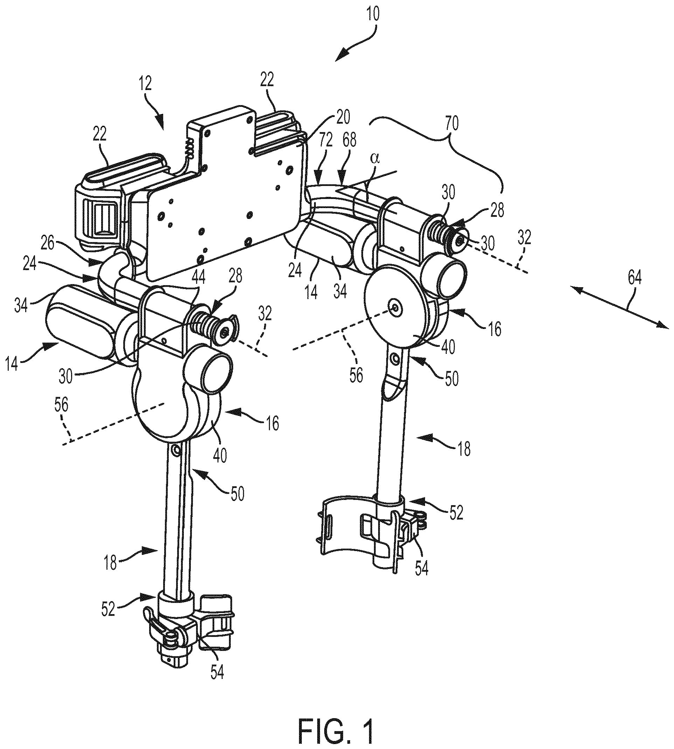

Referring now to the Figures, where the invention will be described with reference to specific embodiments, without limiting same, FIG. 1 illustrates an exemplary adjustable exoskeleton or medical assist device 10. In the exemplary embodiment, adjustable device 10 generally includes a support frame 12, a pair of motor assemblies 14 each respectively associated with a gearbox 16, and a pair of leg supports 18.

Support frame 12 is configured to be disposed about a user's torso or hips and includes a back support 20, one or more power sources 22 (e.g., a battery), a controller (not shown), and a pair of hip supports or support arms 24 extending from back support 20. Back support 20 is configured to rest against a user's back, power source 22 is configured to power motor assembly 14, and the controller is configured to selectively control motor assembly 14 and/or movement of gearbox 16. As used herein, the term controller refers to an application specific integrated circuit (ASIC), an electronic circuit, a processor (shared, dedicated, or group) and memory that executes one or more software
or firmware programs, a combinational logic circuit, and/or other suitable components that provide the described functionality.

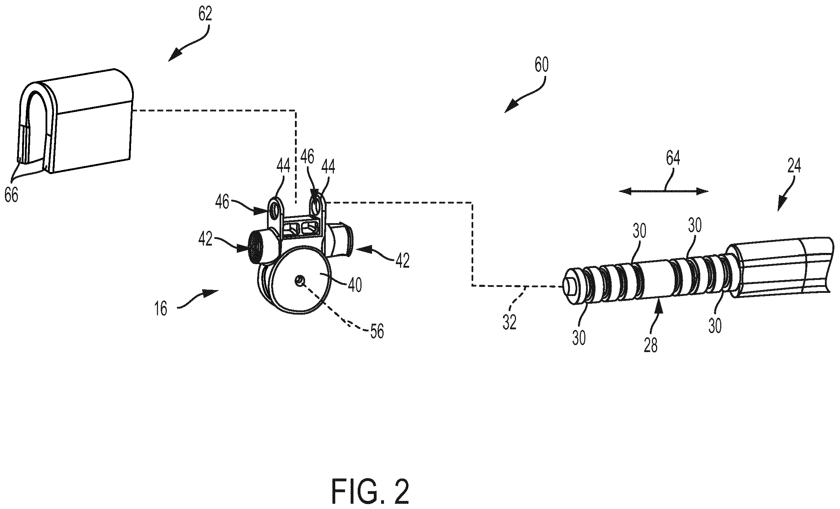

With additional reference to FIG. 2, support arms 24 each include a proximal end 26 and a distal end 28. Proximal end 26 is coupled to back support 20, and distal end 28 includes a plurality of adjustment notches or grooves 30 formed therein. Distal end 28 extends along a longitudinal axis 32 and has a circular or generally circular cross-section.

In the exemplary embodiment, motor assembly 14 includes a housing 34, one or more motors (not shown), and an output shaft (not shown) operatively associated with gearbox 16. Motor assembly 14 is configured to apply a torque to gearbox 16 to selectively rotate leg support 18, thus enabling a user's hip joint to be extended or bent.

With further reference to FIG. 2, gearbox 16 is configured to assist and support a user's hip movement and generally includes a housing 40, a motor assembly aperture 42, an internal gear system (not shown), and pivot flanges 44. Motor assembly aperture 42 is configured to receive at least a portion of motor assembly 14 (e.g., the motor output shaft) such that motor assembly 14 is operably coupled to gearbox 16. The gear system is disposed within housing 40 and is operably coupled to the motor output shaft. Pivot flanges 44 extend outwardly from housing 40 and define apertures 46 configured to receive distal end 28 of support arm 24. As such, gearbox 16 is configured to rotate about longitudinal axis 32 of an associated support arm 24 to facilitate hip flexion and extension while pivot 44 allows for, but does not force, hip adduction and abduction.

In the exemplary embodiment, leg support 18 is configured to support a user's upper leg and includes a proximal end 50, a distal end 52, and a leg clamp 54. Proximal end 50 is coupled to the gear system of gearbox 16 such that leg support 18 is rotatable about an axis 56, and leg clamp 54 is coupled to distal end 52. Leg clamp 54 is configured to connect to a user's leg, for example, by a strap connected directly to clamp 54.

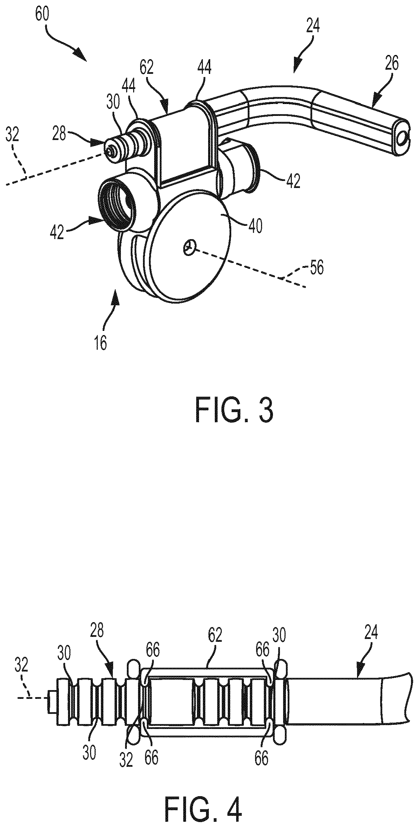

FIGS. 2 and 3 illustrate an exemplary hip joint actuator assembly 60 that generally includes gearbox 16, support arm 24, and a locking mechanism or pivot joint cover 62. FIG. 2 illustrates assembly 60 before assembly, and FIG. 3 illustrates assembly 60 after it has been assembled. Actuator assembly 60 is selectively adjustable in the fore/aft direction illustrated by arrows 64 (FIGS. 3 and 4) to enable medical assist device 10 to conform and fit various sized users, as describe herein in more detail.

During assembly, support arm distal end 28 is inserted into apertures 46 of gearbox pivot flanges 44, and pivot joint cover 62 is inserted over gearbox housing 40 between pivot flanges 44. At this point, as illustrated in FIGS. 2 and 4, inwardly extending flanges 66 of pivot joint cover 62 are seated within grooves 30 formed in support arm distal end 28, which locks and prevents movement of gearbox 16 relative to support arm 24 in the direction of arrows 64 along axis 32.

To adjust medical assist device 10, pivot joint cover 62 may be removed, which enables fore/aft movement of gearbox 16 along support arm distal end 28 in the direction of arrows 64. Once in a desired position, pivot joint cover 62 is again inserted over gearbox housing 40 such that flanges 66 are seated within grooves 30 to lock and prevent further relative movement between gearbox 16 and support arm 24.

In one embodiment, medical assist device 10 may include an angular adjustment 68 to provide further adaptability to various sized users. For example, support arm 24 may include a first portion 70 that is movable inwardly and outwardly relative to a second portion 72. As such, support arm first portion 70 may be oriented in a desired position at an angle 'α' relative to support arm second portion 72. In one embodiment, angle 'α' is between -30° and 60°. In another embodiment, angle 'α' is between 0° and 60°. In one embodiment, angle 'α' is approximately 90° +/- approximately 20°. In another embodiment, angle 'α' is 90° +/- 20°.
assembly 14 and leg support 18 are operably coupled to the gear system of gearbox 16 to transfer rotary motion therebetween. Support arm distal end 28 is inserted into gearbox pivot flanges 44, and pivot joint cover 62 is inserted over a portion of gearbox 16 and support arm distal end 28 such that inwardly extending flanges 66 are seated within grooves 30 to enable rotational movement between gearbox 16 and support arm 28 about axis 32 and to prevent relative movement therebetween along axis 32.

Described herein are systems and methods that provide an adjustable medical assist device. The systems include a support arm with a plurality of grooves that is inserted into a gearbox. A cover is installed over a portion of the support arm and the gearbox such that flanges of the cover are disposed within the groves to prevent movement of the gearbox axially along the support arm. When the cover is removed, the axial positioning of the gearbox along the support arm is adjustable to provide the best fit for the current individual using the medical assist device.

While the invention has been described in detail in connection with only a limited number of embodiments, it should be readily understood that the invention is not limited to such disclosed embodiments. Rather, the invention can be modified to incorporate any number of variations, alterations, substitutions or equivalent arrangements not heretofore described, but which are commensurate with the scope of the invention as defined by the following claims. Additionally, while various embodiments of the invention have been described, it is to be understood that aspects of the invention may include only some of the described embodiments. Accordingly, the invention is not to be seen as limited by the foregoing description.

## Claims

1. An adjustable medical assist device (10) comprising:
a support frame (12) including a support arm (24) extending along a longitudinal axis (32);
a gearbox (16) slidable along the longitudinal axis (32);
a leg support (18) operably coupled to the gearbox (16), the gearbox (16) configured to provide a rotational force to the leg support (18); and
a locking mechanism (62) inserted over a portion of the support arm (24) and the gearbox (16) to prevent movement of the gearbox (16) relative to the support arm (24) along the longitudinal axis (32), the locking mechanism (62) is configured for removal to enable movement of the gearbox (16) relative to the support arm (24) along the longitudinal axis (32),
**characterized in that**
the gearbox (16) includes a housing (40) and a pair of pivot flanges (44) extending outwardly from the housing (40) and each defining an aperture (46) to receive the support arm (24) and rotatably couple the gearbox (16) to the support arm (24) about the longitudinal axis (32),
wherein the locking mechanism (62) is configured for insertion over the support arm (24) between the pair of pivot flanges (44).

2. The device (10) of claim 1, wherein the support arm (24) includes a plurality of grooves (30) formed therein.

3. The device (10) of claim 2, wherein the locking mechanism (62) includes at least one flange (66) that is disposed within at least one groove of the plurality of grooves (30) when the locking mechanism (62) is inserted over the portion of the support arm (24) and the gearbox (16).

4. The device (10) of claim 1, further comprising a motor assembly (14) operatively coupled to the gearbox (16) to transfer rotary motion thereto.

5. The device (10) of claim 4, wherein the gearbox (16) includes a motor assembly aperture (42) to receive at least a portion of the motor assembly (14).

6. The device (10) of claim 1, wherein the support arm (24) includes a first portion (70) and a second portion (72), the first portion (70) selectively adjustable relative to the second portion (72) to orient the first portion (70) at a desired angle relative to the second portion (72).

7. A method of assembling an adjustable medical assist device (10), the method comprising:
providing a support frame (12) including a support arm (24) extending along a longitudinal axis (32);
providing a gearbox (16);
coupling the gearbox (16) to the support arm (24) such that the gearbox (16) is slidable along the support arm (24) along the longitudinal axis (32);
coupling a leg support (18) to the gearbox (16) such that the gearbox (16) is configured to provide a rotational force to the leg support (18); and
inserting a locking mechanism (62) over a portion of the support arm (24) and the gearbox (16) to prevent movement of the gearbox (16) relative to the support arm (24) along the longitudinal axis (32), the locking mechanism (62) configured for removal to enable movement of the gearbox (16) relative to the support arm (24) along the longitudinal axis (32),
**characterized by**
rotatably coupling the gearbox (16) to the support arm (24) about the longitudinal axis (32) by inserting a distal end of the support arm (24) into respective apertures (46) defined by a pair of pivot flanges (44) extending outwardly from a gearbox housing (40),
wherein the locking mechanism (62) is inserted over the support arm (24) between the pair of pivot flanges (44).

## Patentansprüche

1. Einstellbare medizinische Hilfsvorrichtung (10), umfassend:
einen Trägerrahmen (12), der einen Trägerarm (24) aufweist, der sich entlang einer Längsachse (32) erstreckt;
einen Getriebekasten (16), der entlang der Längsachse (32) verschiebbar ist;
einen Beinträger (18), der mit dem Getriebekasten (16) funktional gekoppelt ist, wobei der Getriebekasten (16) derart konfiguriert ist, eine Rotationskraft auf den Beinträger (18) bereitzustellen; und
einen Verriegelungsmechanismus (62), der über einem Abschnitt des Trägerarms (24) und des Getriebekastens (16) eingesetzt ist, um eine Bewegung des Getriebekastens (16) bezüglich des Trägerarms (24) entlang der Längsachse (32) zu verhindern, wobei der Verriegelungsmechanismus (62) zur Entfernung konfiguriert ist, um eine Bewegung des Getriebekastens (16) relativ zu dem Trägerarm (24) entlang der Längsachse (32) zu ermöglichen,
**dadurch gekennzeichnet, dass**
der Getriebekasten (16) ein Gehäuse (40) sowie ein Paar von Drehflanschen (44) aufweist, die sich auswärts von dem Gehäuse (40) erstrecken und jeweils eine Durchbrechung (46) definieren, um den Trägerarm (24) aufzunehmen und den Getriebekasten (16) rotatorisch mit dem Trägerarm (24) um die Längsachse (32) zu koppeln,
wobei der Verriegelungsmechanismus (62) zum Einsetzen über den Trägerarm (24) zwischen dem Paar von Drehflanschen (44) konfiguriert ist.

2. Vorrichtung (10) nach Anspruch 1, wobei der Trägerarm (24) eine Mehrzahl von darin geformten Nuten (30) aufweist.

3. Vorrichtung (10) nach Anspruch 2, wobei der Verriegelungsmechanismus (62) zumindest einen Flansch (66) aufweist, der in zumindest einer Nut der Mehrzahl von Nuten (30) angeordnet ist, wenn der Verriegelungsmechanismus (62) über dem Abschnitt des Trägerarms (24) und des Getriebekastens (16) eingesetzt ist.

4. Vorrichtung (10) nach Anspruch 1, ferner mit einer Motorbaugruppe (14), die funktional mit dem Getriebekasten (16) gekoppelt ist, um eine Rotationsbewegung daran zu übertragen.

5. Vorrichtung (10) nach Anspruch 4, wobei der Getriebekasten (16) eine Motorbaugruppendurchbrechung (42) aufweist, um zumindest einen Anteil der Motorbaugruppe (14) aufzunehmen.

6. Vorrichtung (10) nach Anspruch 1, wobei der Trägerarm (24) einen ersten Abschnitt (70) und einen zweiten Abschnitt (72) aufweist, wobei der erste Abschnitt (70) selektiv bezüglich des zweiten Abschnitts (72) einstellbar ist, um den ersten Abschnitt (70) unter einem gewünschten Winkel bezüglich des zweiten Abschnitts (72) zu orientieren.

7. Verfahren zum Zusammenbauen einer einstellbaren medizinischen Hilfsvorrichtung (10), wobei das Verfahren umfasst, dass:
ein Trägerrahmen (12) bereitgestellt wird, der einen sich entlang einer Längsachse (32) erstreckenden Trägerarm (24) aufweist;
ein Getriebekasten (16) bereitgestellt wird;
der Getriebekasten (16) mit dem Trägerarm (24) so gekoppelt wird, dass der Getriebekasten (16) entlang des Trägerarms (24) entlang der Längsachse (32) verschiebbar ist;
ein Beinträger (18) mit dem Getriebekasten (16) gekoppelt wird, so dass der Getriebekasten (16) derart konfiguriert ist, eine Rotationskraft auf dem Beinträger (18) bereitzustellen; und
ein Verriegelungsmechanismus (62) über einem Abschnitt des Trägerarms (24) und des Getriebekastens (16) eingesetzt wird, um eine Bewegung des Getriebekastens (16) relativ zu dem Trägerarm (24) entlang der Längsachse (32) zu verhindern, wobei der Verriegelungsmechanismus (62) zur Entfernung konfiguriert ist, um eine Bewegung des Getriebekastens (16) relativ zu dem Trägerarm (24) entlang der Längsachse (32) zu ermöglichen,
**dadurch gekennzeichnet, dass**
der Getriebekasten (16) rotatorisch mit dem Trägerarm (24) um die Längsachse (32) gekoppelt ist, indem ein distales Ende des Trägerarms (24) in jeweilige Durchbrechungen (46) eingesetzt wird, die von einem Paar von Drehflanschen (44) definiert sind, die sich von einem Getriebekastengehäuse (40) nach außen erstrecken,
wobei der Verriegelungsmechanismus (62) über dem Trägerarm (24) zwischen dem Paar von Drehflanschen (44) eingesetzt ist.

## Revendications

1. Dispositif d'assistance médicale réglable (10) comprenant :
un cadre de support (12) incluant un bras de support (24) s'étendant le long d'un axe longitudinal (32) ;
une boîte à engrenages (16) capable de coulisser le long de l'axe longitudinal (32) ;
un support de pied (18) fonctionnellement couplé à la boîte à engrenages (16), la boîte à engrenages (16) étant configurée pour appliquer une force de rotation au support de pied (18) ; et
un mécanisme de blocage (62) inséré par-dessus une portion du bras de support (24) et de la boîte à engrenages (16) pour empêcher un mouvement de la boîte à engrenages (16) par rapport au bras de support (24) le long de l'axe longitudinal (32), le mécanisme de blocage (62) étant configuré pour être enlevé et permettre un mouvement de la boîte à engrenages (16) par rapport au bras de support (24) le long de l'axe longitudinal (32),
**caractérisé en ce que**
la boîte à engrenages (16) inclut un boîtier (40) et une paire de brides de pivot (44) s'étendant vers l'extérieur depuis le boîtier (40), chacune définissant une ouverture (46) pour recevoir le bras de support (24) et coupler en rotation la boîte à engrenages (16) au bras de support (24) autour de l'axe longitudinal (32),
dans lequel le mécanisme de blocage (62) est configuré pour être inséré par-dessus le bras de support (24) entre la paire de brides de pivot (44).

2. Dispositif (10) selon la revendication 1, dans lequel le bras de support (24) inclut une pluralité de gorges (30) formées à l'intérieur.

3. Dispositif (10) selon la revendication 2, dans lequel le mécanisme de blocage (62) inclut au moins une bride (66) qui est disposée à l'intérieur d'au moins une gorge de la pluralité de gorges (30) quand le mécanisme de blocage (62) est inséré par-dessus la portion du bras de support (24) et la boîte à engrenages (16).

4. Dispositif (10) selon la revendication 1, comprenant en outre un assemblage moteur (14) fonctionnellement couplé à la boîte à engrenages (16) pour transférer un mouvement de rotation à celle-ci.

5. Dispositif (10) selon la revendication 4, dans lequel la boîte à engrenages (16) inclut une ouverture (42) pour assemblage moteur afin de recevoir au moins une portion de l'assemblage moteur (14).

6. Dispositif (10) selon la revendication 1, dans lequel le bras de support (24) inclut une première portion (70) et une seconde portion (72), la première portion (70) étant sélectivement réglable par rapport à la seconde portion (72) afin d'orienter la première portion (70) à un angle désiré par rapport à la seconde portion (72).

7. Procédé pour assembler un dispositif d'assistance médicale réglable (10), le procédé comprenant les étapes consistant à :
fournir un cadre support (12) incluant un bras de support (24) s'étendant le long d'un axe longitudinal (32) ;
fournir une boîte à engrenages (16) ;
coupler la boîte à engrenages (16) au bras de support (24) de telle façon que la boîte à engrenages (16) est capable de coulisser le long du bras de support (24) le long de l'axe longitudinal (32) ;
coupler un support de pied (18) à la boîte à engrenages (16) de telle façon que la boîte à engrenages (16) est configurée pour appliquer une force de rotation au support de pied (18) ; et
insérer un mécanisme de blocage (62) par-dessus une portion du bras de support (24) et la boîte à engrenages (16) pour empêcher un mouvement de la boîte à engrenages (16) par rapport au bras de support (24) le long de l'axe longitudinal (32), le mécanisme de blocage (62) étant configuré pour être enlevé afin de permettre un mouvement de la boîte à engrenages (16) par rapport au bras de support (24) le long de l'axe longitudinal (32),
**caractérisé par** l'étape consistant à coupler en rotation la boîte à engrenages (16) au bras de support (24) autour de l'axe longitudinal (32) en insérant une extrémité distale du bras de support (24) dans des ouvertures respectives (46) définies par une paire de brides de pivot (44) s'étendant vers l'extérieur depuis un boîtier (40) de la boîte à engrenages,
dans lequel le mécanisme de blocage (62) est inséré par-dessus le bras de support (24) entre la paire de brides de pivot (44).
